# EUROPEAN PATENT SPECIFICATION

(11) **EP 3 727 013 B1**
(45) Date of publication and mention of the grant of the patent: **05.03.2025**
(21) Application number: 18822405.9
(22) Date of filing: 21.12.2018
(51) Int. Cl.: A23J 1/00, A23J 3/34, A23K 20/147, A23L 33/18, A61K 8/00

(54) **PROTEIN HYDROLYSATE AND PROCESS FOR MAKING SUCH**
PROTEINHYDROLYSAT UND VERFAHREN ZU DESSEN HERSTELLUNG
HYDROLYSAT DE PROTÉINES ET SON PROCÉDÉ DE FABRICATION

(30) Priority: 22.12.2017 EP 17210426
(43) Date of publication of application: 28.10.2020
(73) Proprietor: Tessenderlo Group NV, 1050 Brussel (BE)
(72) Inventor: BUSTAMANTE, Pablo César, 3017 Sauce Viejo (AR); GARCÍA, Heber Alejandro, 3017 Sauce Viejo (AR); PEROT, Karina Lorena, 3000 Santa Fe (AR)
(74) Representative: Hoyng Rokh Monegier B.V.
(86) International application number: PCT/EP2018/086693
(87) International publication number: WO 2019/122376

(56) References cited:
- WO-A1-2009/036493
- CN-A- 105 581 964
- GB-A- 2 370 751
- US-A- 3 743 514
- US-A1- 2006 040 033
- US-A1- 2009 269 455

## Description

### TECHNICAL FIELD

The invention relates to a protein hydrolysate and a process for making such protein hydrolysate. In particular the invention relates to a collagen hydrolysate and a process for making such collagen hydrolysate. Further the invention relates to a food, pet food, cosmetic, pharmaceutical or technical application comprising the protein hydrolysate of the present invention and further food, pet food, cosmetic, pharmaceutical or technical constituents/ingredients.

### BACKGROUND

Proteins are important in the food industry, pet food industry, the pharmaceutical industry, the cosmetic and the technical industry as one of the building blocks of most food, pet food, pharmaceutical, cosmetic and technical applications. In food and pet food, proteins provide the nitrogen and the essential amino acids necessary to the growth and maintenance of body tissues. Also in pharmaceutical, cosmetic and technical applications proteins have an important functional role to play.

In many cases, hydrolyzed proteins, or protein hydrolysates are preferred over the non-hydrolyzed proteins, as they are more easily absorbed and digested by the human and animal body.

Protein sources are numerous and proteins can be derived either from animal or from vegetable source.

In many food, pet food, pharmaceutical, cosmetic and technical applications, but also in most production processes of such applications, the ease of use of protein hydrolysates is key. Many protein hydrolysates are available as a dry powder and are not easily dispersible and take long time to dissolve completely in liquids such as water or aqueous solutions. Also lump formation during dissolution often occur and is a real issue in manufacturing processes. There are protein hydrolysates on the market having acceptable dispersibility and dissolving properties (a.o. hydration time and dissolution time), however, when dispersibility and dissolving properties are improved this has a negative effect on the bulk density of the protein hydrolysate, said bulk density becomes too low. One way of improving dispersibility and dissolving properties is by a process known as agglomeration. Known agglomeration processes provide powders with improved dissolving properties, but at the same time have the effect of decreasing the bulk density of such powders. A low bulk density is not desired as it reduces the ease of use, creates problems of dust formation and the like. Higher bulk density also allows to transport more material for a given volume. There is thus a need for providing protein hydrolysates having at the same time improved dissolving properties and improved, i.e. increased/higher, bulk density. The present invention aims to provide such improved protein hydrolysate and a process for making such.

### SUMMARY OF THE INVENTION

The present invention provides for a protein hydrolysate and in particular for a collagen hydrolysate having improved dissolving properties, in particular improved hydration time and dissolution time. Preferably further, the present invention provides for a protein hydrolysate and in particular for a collagen hydrolysate having improved, i.e. higher, bulk density properties.

The present invention relates to a protein hydrolysate characterized in that it has
a. A moisture content of from 4 to 12wt%,
b. A protein content of from 85wt% of more,
c. A hydration time of 7 seconds or less, as determined by "Method A" defined herein, and
d. A dissolution time of 250 seconds or less, as determined by "Method B" defined herein.

Further, the present invention relates to a process to produce a protein hydrolysate characterized in that it comprises the steps of:
a. Incubating a composition comprising protein with a peptidase to obtain a composition comprising protein hydrolysate, and
b. Optionally demineralizing the composition comprising protein hydrolysate, and
c. Adjusting the dry substance content of the composition comprising protein hydrolysate to a dry substance of from 40 to 65% to obtain a concentrated composition comprising protein hydrolysate, and
d. Drying the concentrated composition comprising protein hydrolysate to obtain a dried protein hydrolysate, and
e. Agglomerating the dried protein hydrolysate, and
f. Collecting the protein hydrolysate.

### DETAILED DESCRIPTION

The present invention relates to a protein hydrolysate characterized in that it has
a. A moisture content of from 4 to 12wt%, and
b. A protein content of from 85wt% of more, and
c. A hydration time of 7 seconds or less, and
d. A dissolution time of 250 seconds or less.

In the description of the present invention, the protein may be one or more animal protein or one or more vegetable protein or a mixture thereof. Animal proteins include collagen, whey protein, milk protein, blood protein, insect proteins and the like. Vegetable proteins include cereal protein such as wheat gluten, pea protein, lupine protein, soy protein and the like. Preferably, the protein is animal protein and more preferably the protein is collagen and/or whey protein, more preferably the protein is collagen. Thus more preferably, the present invention relates to a collagen hydrolysate characterized in that it has
a. A moisture content of from 4 to 12wt%, and
b. A protein content of from 85wt% of more, and
c. A hydration time of 7 seconds or less, and
d. A dissolution time of 250 seconds or less.

Collagen is the main component of connective tissues in animals and humans. Collagen consists of chains of amino acids wound together to form triple-helices which in turn form elongated fibrils. It is mostly found in fibrous tissues such as tendons, ligaments and skin. It is also found in bones, teeth, corneas, cartilage, intervertebral discs and blood vessels. Collagen for industrial processing is mostly derived from skin and/or bones. Collagen may be processed to produce gelatin, which is obtained by irreversible, partial hydrolysis of collagen. According to the Food Chemical Codex, gelatin is defined as the product obtained from the acid, alkaline, or enzymatic hydrolysis of collagen, the chief protein component of the of the skin, bones, and connective tissue of animals, including fish and poultry. Gelatin is typically characterized by a gelstrenght and in warm solution by a certain viscosity. This gelatin can be further hydrolyzed to shorter protein chains to produce collagen hydrolysate, losing its ability to form a gel and becoming even soluble in water at ambient temperatures. Hydrolysis can be done by an enzymatic treatment for example.

Preferably the protein hydrolysate of the present invention has a protein content of from 87wt% or more, preferably of from 90wt% or more, more preferably of from 95wt% or more, yet even more preferably of from 98wt% or more, most preferably up to 99wt% of protein on dry basis (wt% db).

The hydration time (H) represents the time needed for the protein hydrolysate to fully sink to the bottom of a recipient filled with water, it is measured according to method A described further below in this description. Preferably the protein hydrolysate of the present invention has a hydration time of 6 seconds or less, more preferably of 5 seconds or less, even more preferably of 4 seconds or less, yet even more preferably of 3 seconds or less, yet even more preferably of 2 seconds or less, most preferably of 1 second or less.

The dissolution time (D) means the time needed for substantially all the particles of the protein hydrolysate to be dissolved in water. It is measured according to method B described further below in this description. Preferably the dissolution time is of 200 seconds or less, more preferably of 190 seconds or less, more preferably of 180 seconds or less, more preferably of 170 seconds or less, even more preferably of 160 seconds or less, yet even more preferably of 150 seconds or less, yet even more preferably of 140 seconds or less, yet even more preferably of 130 seconds or less, yet even more preferably of 120 seconds or less, yet even more preferably of 110 seconds or less, most preferably of 100 seconds or less. Further preferably, the protein hydrolysate of the present invention forms a clear solution when dissolved in sufficient water in that no more particles are visible with the naked eye.

The protein hydrolysate of the present invention is dry, i.e. it has a moisture content of from 4 to 12wt%, preferably from 4 to 10wt%, more preferably from 5 to 8wt%. Preferably the protein hydrolysate is dry and in powder form, meaning that it is free flowing and does substantially not form any lumps upon handling or storage.

Preferably, the protein hydrolysate of the present invention has a bulk density (or loose density, non-tapped density) of 240g/l or higher, preferably of 250g/l or higher, more preferably of 260 or higher, more preferably of 270g/l or higher, more preferably of 280 g/l or higher, more preferably of 290 g/l or higher, more preferably of 300 g/l or higher, more preferably of 310 g/l or higher, more preferably of 320 g/l or higher, more preferably of 330 g/l or higher, more preferably of 340 g/l or higher, more preferably of 350 g/l or higher, more preferably of from 350g/l to 400g/l, preferably of 360g/l or higher, more preferably of from 360g/l to 400g/l, more preferably of 370g/l or higher, more preferably of from 370g/l to 400g/l. In a preferred embodiment, the protein hydrolysate of the present invention has a bulk density of 340 g/l or higher, more preferably of 350 g/l or higher, more preferably of from 350g/l to 400g/l, preferably of 360g/l or higher, more preferably of from 360g/l to 400g/l, more preferably of 370g/l or higher, more preferably of from 370g/l to 400g/l.

Preferably, the protein hydrolysate of the present invention is further characterized in that it has an average molecular weight of from 300 to 15000 Da, preferably from 500 to 10000Da, preferably from 600 to 10000 Da, preferably from 700 to 10000 Da, preferably from 800 to 10000 Da, preferably from 900 to 10000 Da, preferably from 1000 to 10000 Da, preferably from 1500 to 10000 Da, preferably from 1700 to 9000 Da, preferably from 2000 to 7000 Da, preferably of from 2000 to 5000 Da, more preferably of from 2500 to 4500 Da, even more preferably of from 2700 to 4000 Da, yet even more preferably of from 2800 to 3800 Da (average molecular weight (Mw) measured by gel permeation chromatography (Size exclusion chromatography using linear polymers such as polystyrene sulfonates or collagen chain fragments of different molecular weights as calibration vehicles)).

Preferably, the protein hydrolysate of the present invention is further characterized in that it has a d50 particle size of from 100 to 1000 µm, preferably from 110 to 800 µm, more preferably from 120 to 600 µm, even more preferably from 130 to 500 µm, yet even more preferably from 140 to 400µm, yet even more preferably from 150 to 300µm, yet even more preferably from 160 to 250µm, yet even more preferably from 170 to 250µm, most preferably from 170 to 200 µm. In the present description d50 means the particle size where 50% of the volume fraction of the particles is larger than the indicated d50 value, and 50% is smaller than the d50 indicated value. It is measured by laser diffraction on a Beckman Coulter particle size analyzer using standard software.

Preferably the protein hydrolysate of the present invention is in agglomerated form, thus preferably the present invention relates to an agglomerated protein hydrolysate as described herein, more preferably to an agglomerated collagen hydrolysate as described herein. Agglomeration means that particles have been agglomerated together to form bigger particles, which is advantageous to reduce the dissolution time and hydration time. Preferably agglomeration is done as described further in this description.

The present invention further relates to a process to produce a protein hydrolysate characterized in that it comprises the steps of:
a. Incubating a composition comprising protein with a peptidase to obtain a composition comprising protein hydrolysate, and
b. Optionally demineralizing the composition comprising protein hydrolysate, and
c. Adjusting the dry substance content of the composition comprising protein hydrolysate to a dry substance of from 40 to 65% to obtain a concentrated composition comprising protein hydrolysate, and
d. Drying the concentrated composition comprising protein hydrolysate to obtain a dried protein hydrolysate, and
e. Agglomerating the dried protein hydrolysate, and
f. Collecting the protein hydrolysate.

In step a. of the process of the present invention a composition comprising protein is incubated with a peptidase to obtain a composition comprising protein hydrolysate. For example, the composition may be an aqueous composition comprising from 45 to 97wt%, preferably from 50 to 95wt%, more preferably from 60 to 90wt%, even more preferably from 65 to 80wt%, yet even more preferably from 70 to 75wt% of water. Said composition comprising protein may be an aqueous composition wherein protein is freely available, dissolved and/or not dissolved, such as for example an aqueous composition comprising gelatin and/or collagen. Advantageously said composition comprising gelatin may be obtained from a typical industrial process to produce gelatin from collagen containing material such as animal skin, for example beef hide and/or pork skin and/or fish skin; animal bones, such as pig bones and/or beef bones and/or fish scales and bones and/or poultry bones. Alternatively, the composition of step a. may be a composition comprising collagen such as animal skin, for example beef hide and/or pork skin and/or fish skin; animal bones, such as pig bones and/or beef bones and/or fish scales and bones and/or poultry bones.

Incubation is done with one or more peptidases, which may be endopeptidases, exopeptidases or a combination of two or more thereof. Suitable peptidases are known to the skilled person and depend on the specific protein or protein mixture to be hydrolyzed. A broad spectrum of proteolytic enzymes can be used, for example, but not limited to, proteolytic enzymes of vegetable origin like papain, bromelaine, ficin; of animal origin like pepsin; and/or microbial enzymes of Bacillus Licheneformis, Aspergillus or Bacillus Subtilis in general. Commercial enzymes are available under the trademarks like for example Alcalase^{®}, Neutrase^{®}, Corolase^{®}, Protex^{®}, Flavourzyme^{®}. Preferably, the protein comprises gelatin and/or collagen and the one or more peptidases are suitable to hydrolyze said protein.

Preferably the incubation conditions are chosen such that hydrolysis is done until the protein hydrolysate has an average molecular weight (Mw) of from 300 to 15000 Da, preferably from 500 to 10000Da, preferably from 600 to 10000 Da, preferably from 700 to 10000 Da, preferably from 800 to 10000 Da, preferably from 900 to 10000 Da, preferably from 1000 to 10000 Da, preferably from 1500 to 10000 Da, preferably from 1700 to 9000 Da, preferably from 2000 to 7000 Da, preferably of from 2000 to 5000 Da, more preferably of from 2500 to 4500 Da, even more preferably of from 2700 to 4000 Da, yet even more preferably of from 2800 to 3800 Da (average molecular weight (Mw) measured by gel permeation chromatography (Size exclusion chromatography using linear polymers such as polystyrene sulfonates or collagen chain fragments of different molecular weights as calibration vehicles)).

The skilled person will be able to determine the suitable pH of incubation, depending on the enzyme or enzyme cocktail that is used. Preferably, the pH will be of from 5 to 8, more preferably from 6 to 7.

Also the incubation time depends on the enzyme used and the enzyme:substrate ratio. Preferably, the incubation time will be of from 1 to 10 hours, more preferably from 2 to 7 hours, even more preferably from 2 to 5 hours, yet even more preferably from 2 to 4 hours.

The incubation temperature also depends on the enzyme or enzyme cocktail used. Incubation temperature is preferably from 30 to 80°C, more preferably from 40 to 70°C, and even more preferably from 50 to 70°C.

A demineralization step b. may be performed after the incubation step a. Preferably during such demineralization step charged components are at least partially removed. Demineralization may be done using techniques known in the art such as ion exchange chromatography, reversed osmosis, membrane filtration. The charged components that may be removed include salt anions and cations such as for example calcium cations, magnesium cations, sodium cations, ammonium cations and chloride anions, sulfate anions, phosphate anions, nitrate anions and the like. A demineralization step is particularly preferred when the composition comprising protein is a composition comprising collagen such as animal skin, for example beef hide and/or pork skin and/or fish skin; animal bones, such as pig bones and/or beef bones and/or fish scales and bones and/or poultry bones.

In step c. of the process of the present invention, the dry substance of the composition comprising protein hydrolysate is adjusted, preferably increased, until a dry substance of from 40 to 65%, preferably of from 45 to 60%, more preferably of from 50 to 55% is obtained. Several techniques may be used to increase the dry substance, for example by removing water from the composition or by back-mixing of concentrated composition obtained in step c. with the composition of step a. or b., or addition of dry protein hydrolysate to the composition, preferably by removing water from the composition. Techniques for removing water from the composition include evaporation, nanofiltration and the like. Preferably evaporation is done by passing the composition through a vacuum evaporator or through a heat exchanger, more preferably a multistage vacuum evaporator or by using falling film evaporators or rising film evaporators. Nanofiltration is preferably performed using a membrane having a molecular weight cut-off of 500 Da or lower, preferably 400 Da or lower, more preferably of 300 Da or lower, even more preferably of 250 Da or lower. The molecular weight cut-off of a membrane refers to the lowest molecular weight particle (in daltons (Da)) for which 90% of the particles is retained by the membrane.

In step d. of the process, the composition comprising protein hydrolysate is dried. Thus in step d., a dried protein hydrolysate is obtained. Drying may be done using known techniques in the art such as for example freeze drying, drum drying, spray drying, flash drying. Preferably drying is done by spray drying using a nozzle system, as this is advantageous for providing improved powder characteristics, in particular improved bulk density. Additionally, product characteristics are more constant, and this drying technology can be fully automated and continuous and is suitable for heat-sensitive products such as proteins. Drying is preferably done until a moisture content of 2 to 12wt%, preferably 3 to 12wt%, more preferably 4 to 12wt%, yet even more preferably 3 to 9wt%, yet even more preferably 4 to 8wt%, yet even more preferably 5 to 7wt% is obtained.

In step e. of the process, the dried protein hydrolysate is agglomerated. During agglomeration, dried protein hydrolysate particles are stick to each other and form particle clusters, aggregates or agglomerates. Thus the particle size of the product is enlarged through agglomeration of smaller product particles. Agglomeration may be done by using pressure, extrusion, rewetting, spray-bed drying, steam jet, heat (also called sintering). Preferably agglomeration is done using rewetting, i.e. using a wet component or binder which acts like a glue to stick particles together, on a fluidized bed. The binder may be water of ethanol and the like, preferably it is water. In particular when water is used as a binder, the moisture content of the product is slightly increased during agglomeration. Agglomeration is known to reduce bulk density of powders. As mentioned above, bulk density properties of protein hydrolysates is typically poor, i.e. low. The inventors have found however, that as a result of the process of the present invention, not only protein hydrolysate with good dissolving properties are obtained but also with improved, i.e. higher, bulk density properties, in particular due to the combination of steps c., d. and e. of the process.

In view of the agglomeration step thus, preferably the process of the present invention relates to a process to produce an agglomerated protein hydrolysate, wherein step f. comprises collecting the agglomerated protein hydrolysate.

After agglomeration, the protein hydrolysate, or agglomerated protein hydrolysate, is collected and may be further packaged, stored, transported and sold for use in for example food, pet food, pharmaceutical, cosmetic and technical applications.

A food application includes, but is not limited to, bakery products, such as bread, cakes, sponge cakes, cereal bars and the like; dairy products, such as yogurts, dairy desserts and the like; beverages, such as smoothies, nutrition beverages, protein beverages, meal replacement beverages, waters, fruit juices, fruit concentrates and the likes. Preferably the food application is a beverage application. The protein hydrolysate of the present invention may also be used in dry mixes to make instant beverages for examples, wherein the dry mix is mixed with water by the consumer before consumption.

Food constituents depend on the particular food composition and include, but are not limited to, carbohydrates, such as sweeteners, starches, hydrocolloids, bulking agents; fats and oils; aromas; flavorings; vitamins; minerals; colorants and the like. A pet food application includes, but is not limited to, dry and wet pet food, in particular treats. Pet food constituents include, but are not limited to, carbohydrates, fats and oils, flavorings, colorants, vitamins, minerals, and the like.

A pharmaceutical application includes, but is not limited to, nutraceutical compositions, food supplements, tablets and the like.

Pharmaceutical constituents include, but are not limited to, carbohydrates, in particular bulking agents, sweeteners, vitamins, and the like.

A cosmetic application includes, but is not limited to, creams, lotions for skin care, shampoos and lotions for hair care and the like. Preferably the cosmetic composition is a cream or lotion for skin care.

Cosmetic application include, but are not limited to, bulking agents, fats and oils, coloring agents, aromas and the like.

A technical application includes, but is not limited to, electroplating, oil drilling and the like.

The present invention further relates to the use of the protein hydrolysate according to the present invention in food, pet food, pharmaceutical, cosmetic or technical applications.

The methods of measurement referred to in the present description are explained below:
Hydration and dissolution time are measured according to following methods:

### Method A/B:

Sample: weigh a sample of 2.5g protein hydrolysate.

A 400ml beaker is filled with 300ml tap water having a temperature of from 15 to 20°C.

The protein hydrolysate sample is added all at once in one go to the water without stirring.

Start chronometer and observe behavior of the protein hydrolysate.

After 1 minute, stir 3x with a tablespoon.

### Evaluation :

Method A - Hydration time (H) or wettability: the time needed for the whole protein hydrolysate sample to sink to the bottom of the beaker filled with water such that no more lumps are floating on the surface of the water, corresponds to the hydration time of that sample.
- If the sample sinks to the bottom immediately after addition to the water, H = 0 seconds
- If the sample does not immediately sink to the bottom, note the number of seconds until the sample sinks to the bottom of the beaker until no more lumps are floating on the surface of the water, and H corresponds to said number of seconds.

Method B - Dissolution time (D) or dissolution rate: the time needed for the whole protein hydrolysate sample to be dissolved in the water, meaning no visible particles remain in the solution:
- If the sample is dissolved without the need of stirring, D = 0 seconds
- If stirring after 1 minute is needed for the sample to dissolve, D corresponds to the number of seconds needed for the sample to dissolve and is computed from the start of the chronometer. From the start of the chronometer, the sample is stirred 3x with a table spoon every minute until dissolved.

Bulk density is measured according to following method:
- An empty and clean metered cylinder of 100ml is weighed (W0)
- The cylinder is filled with sample until 100ml
- The filled cylinder is weighed (W1) and the sample weight is calculated (W1-W0)
- The bulk density corresponds to the sample weight divided by 100ml and expressed in g/l.

The invention will be further illustrated in the non-limiting examples.

### Example 1

The following example allows to produce a collagen hydrolysate according to the invention:
A gelatin solution of 27% dry substance is obtained after thermal hydrolysis of beef skin collagen, purified by cellulose filtration and deionization using ion exchange and concentrated in a multistage evaporator is further hydrolyzed enzymatically. Temperature is at 58°C and pH is adjusted using Ca(OH) 2 till pH of 6.2 and further by 50% NaOH to pH 6.7. Alcalase 2.4 FG Novozyme enzyme solution is added at E/S ratio of 0.1% w/w. Reaction is stopped after 5h by passing over an heat exchanger at 85°C for 10min.

The reaction mixture containing the collagen hydrolysate is further filtered and deionized using cation Lanxcess S1468 and anion resins Lanxcess VPOC1072. The purified solution is concentrated in a multistage evaporator till 50% dry substance, pasteurized at 78°C using a heat exchanger and spray dried using a nozzle system followed by agglomeration in a series of fluidized beds.

The agglomerated collagen hydrolysate is collected and may now be analyzed using measurement methods as described above in this description.

## Claims

1. A protein hydrolysate **characterized in that** it has
a. A moisture content of 4 to 12%,
b. A protein content of from 85wt% or more,
c. A hydration time of 7 seconds or less, as determined by "Method A" defined herein, and
d. A dissolution time of 250 seconds or less, as determined by "Method B" defined herein.

2. The protein hydrolysate of claim 1, **characterized in that** it has a bulk density of 240 g/l or higher.

3. The protein hydrolysate of claims 1 or 2 **characterized in that** the protein hydrolysate is collagen hydrolysate.

4. The protein hydrolysate of any one of the previous claims further **characterized in that** it has an average molecular weight of from 300 to 15000 Da.

5. The protein hydrolysate of any one of the previous claims further **characterized in that** it has a d50 particle size of from 100 to 1000 µm.

6. The protein hydrolysate of any one of the previous claims further **characterized in that** the protein hydrolysate is agglomerated.

7. A process to produce a protein hydrolysate **characterized in that** it comprises the steps of:
a. Incubating a composition comprising protein with a peptidase to obtain a composition comprising protein hydrolysate, and
b. Optionally demineralizing the composition comprising protein hydrolysate, and
c. Adjusting, preferably increasing, the dry substance content of the composition comprising protein hydrolysate to a dry substance of from 40 to 65% to obtain a concentrated composition comprising protein hydrolysate, and
d. Drying the concentrated composition comprising protein hydrolysate to obtain a dried protein hydrolysate, and
e. Agglomerating the dried protein hydrolysate, and
f. Collecting the protein hydrolysate.

8. The process of claim 7 **characterized in that** drying in step d. is done by spray-drying, freeze drying, or drum drying, preferably by spray-drying.

9. The process of any one of claims 7 to 8, **characterized in that** increasing the dry substance in step c. is performed by evaporation or by nanofiltration.

10. The process of claim 9, wherein nanofiltration is performed with a membrane having a molecular weight cut-off of 500 Da or lower.

11. The process of any one of claims 7 to 10, further **characterized in that** the protein is gelatin and is derived from beef hide and/or from pork skin.

12. Use of the protein hydrolysate according to anyone of claims 1 to 6 in food, pet food, pharmaceutical, cosmetic or technical applications.

## Patentansprüche

1. Proteinhydrolysat, **dadurch gekennzeichnet, dass** es aufweist
a Einen Feuchtigkeitsgehalt von 4 bis 12 %,
b. Ein Proteingehalt von 85 Gew.-% oder mehr,
c. Eine Hydratationszeit von 7 Sekunden oder weniger, wie durch das "Verfahren A" bestimmt, das hierin definiert ist, und
d. Eine Auflösungszeit von 250 Sekunden oder weniger, wie durch das "Verfahren B" bestimmt, das hierin definiert ist.

2. Proteinhydrolysat nach Anspruch 1, **dadurch gekennzeichnet, dass** es eine Schüttdichte von 240 g/l oder höher aufweist.

3. Proteinhydrolysat nach Anspruch 1 oder 2, **dadurch gekennzeichnet, dass** das Proteinhydrolysat Kollagenhydrolysat ist.

4. Proteinhydrolysat nach einem der vorstehenden Ansprüche, ferner **dadurch gekennzeichnet, dass** es ein durchschnittliches Molekulargewicht von 300 bis 15.000 Da aufweist.

5. Proteinhydrolysat nach einem der vorstehenden Ansprüche, ferner **dadurch gekennzeichnet, dass** es eine d50-Partikelgröße von 100 bis 1000 µm aufweist.

6. Proteinhydrolysat nach einem der vorstehenden Ansprüche, ferner **dadurch gekennzeichnet, dass** das Proteinhydrolysat agglomeriert wird.

7. Prozess zum Produzieren eines Proteinhydrolysats, **dadurch gekennzeichnet, dass** es die Schritte umfasst:
a. Inkubieren einer Zusammensetzung, umfassend Protein, mit einer Peptidase, um eine Zusammensetzung zu erhalten, umfassend Proteinhydrolysat, und
b. Optionales Entmineralisieren der Zusammensetzung, umfassend Proteinhydrolysat, und
c. Einstellen, vorzugsweise Erhöhen, des Trockensubstanzgehalts der Zusammensetzung, umfassend Proteinhydrolysat, auf eine Trockensubstanz von 40 bis 65 %, um eine konzentrierte Zusammensetzung zu erhalten, umfassend Proteinhydrolysat, und
d. Trocknen der konzentrierten Zusammensetzung, umfassend Proteinhydrolysat, um ein getrocknetes Proteinhydrolysat zu erhalten, und
e. Agglomerieren des getrockneten Proteinhydrolysats und
f. Sammeln des Proteinhydrolysats.

8. Prozess nach Anspruch 7, **dadurch gekennzeichnet, dass** das Trocknen in Schritt d. durch Sprühtrocknung, Gefriertrocknung oder Walzentrocknung, bevorzugt durch Sprühtrocknung, erfolgt.

9. Prozess nach einem der Ansprüche 7 bis 8, **dadurch gekennzeichnet, dass** das Erhöhen der Trockensubstanz in Schritt c. durch Verdampfung oder durch Nanofiltration durchgeführt wird.

10. Prozess nach Anspruch 9, wobei die Nanofiltration mit einer Membran durchgeführt wird, die eine Molekulargewichtsgrenze von 500 Da oder weniger aufweist.

11. Prozess nach einem der Ansprüche 7 bis 10, ferner **dadurch gekennzeichnet, dass** das Protein Gelatine ist und aus Rinderhaut und/oder aus Schweineschwarte gewonnen wird.

12. Verwendung des Proteinhydrolysats nach einem der Ansprüche 1 bis 6 in Lebensmitteln, Tiernahrung, pharmazeutischen, kosmetischen oder technischen Anwendungen.

## Revendications

1. Hydrolysat de protéines **caractérisé en ce qu'**il présente
a. Une teneur en humidité de 4 à 12 %,
b. Une teneur en protéines de 85 % en poids ou plus,
c. Un temps d'hydratation de 7 secondes ou moins, tel que déterminé par la « Méthode A » définie ici, et
d. Un temps de dissolution de 250 secondes ou moins, tel que déterminé par la « Méthode B » définie ici.

2. Hydrolysat de protéines selon la revendication 1, **caractérisé en ce qu'**il présente une masse volumique apparente de 240 g/l ou plus.

3. Hydrolysat de protéines selon les revendications 1 ou 2, **caractérisé en ce que** l'hydrolysat de protéines est un hydrolysat de collagène.

4. Hydrolysat de protéines selon l'une quelconque des revendications précédentes, en outre **caractérisé en ce qu'**il présente une masse moléculaire moyenne de 300 à 15 000 Da.

5. Hydrolysat de protéines selon l'une quelconque des revendications précédentes, en outre **caractérisé en ce qu'**il présente une taille de particule d50 de 100 à 1000 µm.

6. Hydrolysat de protéines selon l'une quelconque des revendications précédentes, en outre **caractérisé en ce que** l'hydrolysat de protéines est aggloméré.

7. Procédé de production d'un hydrolisat de protéines **caractérisé en ce qu'**il comprend les étapes consistant à :
a. Incuber une composition comprenant des protéines avec une peptidase pour obtenir une composition comprenant un hydrolysat de protéines, et
b. Éventuellement déminéraliser la composition comprenant un hydrolysat de protéines, et
c. Ajuster, de préférence augmenter, la teneur en matière sèche de la composition comprenant un hydrolysat de protéines à une matière sèche comprise entre 40 et 65 % pour obtenir une composition concentrée comprenant un hydrolysat de protéines, et
d. Sécher la composition concentrée comprenant un hydrolysat de protéines pour obtenir un hydrolysat de protéines séché, et
e. Agglomérer l'hydrolysat de protéines séché, et
f. Recueillir l'hydrolysat de protéines.

8. Procédé selon la revendication 7, **caractérisé en ce que** le séchage à l'étape d. est effectué par pulvérisation, lyophilisation ou séchage en tambour, de préférence par pulvérisation.

9. Procédé selon l'une quelconque des revendications 7 à 8, **caractérisé en ce que** l'augmentation de la matière sèche à l'étape c. est réalisée par évaporation ou par nanofiltration.

10. Procédé selon la revendication 9, dans lequel une nanofiltration est effectuée avec une membrane ayant un seuil de masse moléculaire de 500 Da ou inférieur.

11. Procédé selon l'une quelconque des revendications 7 à 10, en outre **caractérisé en ce que** la protéine est de la gélatine et est dérivée de la peau de bovin et/ou de porc.

12. Utilisation de l'hydrolysat de protéines selon l'une quelconque des revendications 1 à 6 dans des applications alimentaires, alimentaires destinées aux animaux de compagnie, pharmaceutiques, cosmétiques ou techniques.
